# EUROPÄISCHE PATENTANMELDUNG

(11) **EP 1 666 619 A1**
(43) Veröffentlichungstag der Anmeldung: **07.06.2006**
(21) Anmeldenummer: 04028650.2
(22) Anmeldetag: 03.12.2004
(51) Int. Cl.: C22C 19/07, A61K 6/04

(54) **Kohlenstofffreie Kobalt-Basislegierung**

(71) Anmelder: Amann Girrbach GmbH, 75177 Pforzheim (DE)
(72) Erfinder: Pässler, Klaus, Dr., 75175 Pforzheim (DE)
(74) Vertreter: Frank, Gerhard

(57) **Zusammenfassung**

Eine Kohlenstofffreie Kobalt-Basis-Legierung zur Verwendung in der Dentalprothetik weist folgenden Gehalte an Legierungsbestandteilen auf:

| | | | |
|---|---|---|---|
| Cr | von | 30,0 m% | bis 34,0 m% |
| Mo | von | 4,0 m% | bis 7,0 m% |
| Si | von | 1,0 m% | bis 1,5 m% |
| N | von | 0,1 m% | bis 0,3 m% |
| Nb | von | 0,3 | bis 0,5 m% |
| Mn | von | 0,2 | bis 0,6 m% |
| W | von | 0,1 | bis 0,8 m% |
| Rest Co. | | | |

Insbesondere durch den Zusatz von W wird eine in wesentlichen Bereichen erhebliche Verbesserung der für die Dentalprothetik entscheidenden Parameter (Dehngrenze, Zugfestigkeit, E-Modul) erzielt. Weiterhin wird auch ein verbesserter Korrosionsschutz durch den Zusatz von Mangan als Desoxidationsmittel erreicht.

## Beschreibung

### Technischer Hintergrund

CoCrMo-Legierungen werden unter anderem in der Chirurgie für die Herstellung von Implantaten und in der Zahnheilkunde für fest sitzende und herausnehmbare Prothesen eingesetzt. Um entsprechend hohe mechanische Eigenschaften und ein gutes Schmelzverhalten der Legierungen zu erreichen, kann diesen Kohlenstoff zugesetzt werden (z.B. DE 22 25 577 C3, DE 40 28 870 C2).

Mit dem Laserschweißverfahren, wie es zum Beispiel bei modern ausgerüsteten zahntechnischen Laboratorien üblich ist, ist es möglich, Verbunde und Reparaturen von Implantaten und Prothesen mit hoher Verbundfestigkeit dauerhaft und biokompatibel herzustellen. Beim Laserschweißen Kohlenstoff-haltiger Legierungstypen treten allerdings in den Laserschweißnähten Karbitausscheidungen auf, die oft zu Brüchen und somit zum Ausfall und Versagen der Prothesen führen. Legierungen mit Kohlenstoffzusatz sind hierfür somit nicht geeignet.

Neben der Verbesserung der mechanischen Eigenschaften u.a. auch von CoCrMo-Legierungen hat Kohlenstoff auch eine metallurgische Funktion beim Schmelzprozess, und zwar die der Desoxidation, d.h., Kohlenstoff wirkt einer übermäßigen Sauerstoffaufnahme beim Aufschmelzen von Dentallegierungen entgegen. Zu hohe Sauerstoffgehalte können sich auch in CoCrMo-Legierungen auf die Korrosionsfestigkeit und somit auf die Biokompatibilität auswirken.

### Stand der Technik

Eine kohlenstofffreie Kobalt-Basislegierung für die Dentalprothetik ist aus der DE 198 45 638 C1 bekannt. Sie besitzt einen Cr-Gehalt von 25 bis 35 m%, einen Mo-Gehalt von 4 bis 11 m%, einen Si-Gehalt von 0,5 bis 2,1 m%, einen Gehalt mindestens eines Elementes der fünften Nebengruppe von 0,5 bis 3,0 m%, und einen N-Gehalt von 0,2 bis 0,5 %; der Rest besteht aus Co. Diese Legierung ist im Gegensatz zu den oben genannten kohlenstofffrei, dies bedeutet, dass der Kohlenstoffanteil maximal 0,02 m% beträgt.

Bei der bekannten Legierung wurde festgestellt, dass durch den Zusatz mindestens eines Elementes der fünften Nebengruppe (bevorzugt V, Ta, Nb) die mechanischen Eigenschaften der Legierung verbessert werden. Diese Elemente sind jedoch relativ teuer. Die erzielten mechanischen Eigenschaften reichen auch nicht in allen Fällen zur Erarbeitung qualitativ hochwertigster Dentalprothetik aus.

### Darstellung der Erfindung

Aufgabe der Erfindung ist es, die mechanischen Eigenschaften bei reduzierten Materialkosten zu verbessern.

Erfindungsgemäß wird diese Aufgabe durch die Merkmale des Patentanspruchs 1 gelöst.

Der Grundgedanke der Erfindung ist darin zu sehen, den (kostspieligen) Anteil der Elemente der fünften Nebengruppe zurück zu drängen und durch den Zusatz von W (nur etwa 5% des Preises eines der Elemente der fünften Nebengruppe) dennoch eine in wesentlichen Bereichen erhebliche Verbesserung der für die Dentalprothetik entscheidenden Parameter (Dehngrenze, Zugfestigkeit, E-Modul) zu erzielen.

Es wurde weiterhin festgestellt, dass sich bei der erfindungsgemäßen kohlenstofffreien Legierung auch ein verbesserter Korrosionsschutz durch den Zusatz von Mangan als Desoxidationsmittel erreichen lässt, wobei ein günstiger Mn-Gehalt bei ca. 0,4 m% liegt.

Weitere vorteilhafte Ausgestaltungen sind den Unteransprüchen zu entnehmen.

Die aus der Legierung herzustellenden Teile werden in der Regel durch Aufschmelzen von Gussrohlingen und anschließenden Abguss in zahntechnisch vorbereitete Formen hergestellt. Die Legierungszusammensetzung wird so gewählt, dass der Zeitpunkt des Abgusses durch ein kurzes Spiegeln der Schmelzoberfläche angezeigt wird. Gussfehler wie Lunker oder Grobkornbildung durch zu heißes Gießen oder Kaltgüsse, d.h. kein vollständiges Ausfließen der Gusskörper werden vermieden.

### Beschreibung eines bevorzugten Ausführungsbeispiels

Die chemische Zusammensetzung in m% der vorgeschlagenen nickel- und kohlenstofffreien Kobalt-Basislegierung ist aus folgenden beispielhaften Angaben zu entnehmen:

| Cr | Mo | Si | W | Nb | Mn | N | Co |
|---|---|---|---|---|---|---|---|
| 32 | 6 | 1,3 | 0,6 | 0,45 | 0,4 | 0,30 | Rest |

Für die großtechnische Herstellung vorliegender Legierung wird ein Stranggussverfahren vorgesehen. Die schmelzmetallurgische Weiterverarbeitung der Legierung zu dentalen Gussskeletten kann mit allen in der Zahntechnik für das Schmelzen von CoCrMo-Legierungen vorgesehenen Schmelztechnologien durchgeführt werden.

Das Schmelzverhalten vorliegender Legierung wird durch folgenden Temperaturbereich bestimmt:
Solidus: 1346 °C
und
Liquidus: 1388 °C

Die oben angegebene Legierung weist folgende mechanische Eigenschaften auf:

| Dehngrenze | Zugfestigkeit | E-Modul | Bruchdehnung | Härte |
|---|---|---|---|---|
| Rp 0,2 %(Mpa) | Rm (Mpa) | E (Mpa) | A5 (%) | HV 10 |
| 740 | 940 | 225000 | 6 | 350 |

Von besonderer Bedeutung gegenüber der DE 198 45 638 C1 ist hierbei die Erhöhung des Wertes für den E-Modul auf 225000 Mpa.

Wichtig ist die Korrosionsbeständigkeit und die Biokompatibilität einer Dentallegierung.

Die vorliegende Legierung wurde entsprechend den geltenden Bestimmungen für Dentallegierungen auf ihre Korrosionsbeständigkeit nach
DIN En ISO 10721: 2002
"Static Immersion Test" und auf ihre Zellvitalität nach
ISO 10993 - 5:1999
"biological evalnation of medical devices" untersucht.

Diese Untersuchungen zeigten für die erfindungsgemäße Legierung ausgezeichnete Korrosionsbeständigkeit und keine zytotoxischen Effekte.

Der Stickstoffgehalt sollte über 0,15 m% liegen. Stickstoff stabilisiert die Gammaphase und bewirkt die ausgezeichneten mechanischen Eigenschaften vorliegender Legierung. Die Elemente Si und Mn bewirken in den angegebenen Zusatzmengen ein sehr gutes Schmelz- und -Gießverhalten. Dentale Gussskelette fließen problemlos aus.

## Patentansprüche

1. Kohlenstofffreie Kobalt-Basis-Legierung zur Verwendung in der Dentalprothetik mit folgenden Gehalten:
| | |
|---|---|
| Cr | von 30,0 m% bis 34,0 m% |
| Mo | von 4,0 m% bis 7,0 m% |
| Si | von 1,0 m% bis 1,5 m% |
| N | von 0,1 m% bis 0,3 m% |
| Nb | von 0,3 bis 0,5 m% |
| Mn | von 0,2 bis 0,6 m% |
| W | von 0,1 bis 0,8 m% Rest Co. |

2. Kohlenstofffreie Kobalt-Basis-Legierung nach Anspruch 1, **gekennzeichnet durch** einen Gehalt von
| | |
|---|---|
| Cr | 32,0 m% |
| Mo | 6,0 m% |
| Si | 1,3 m% |
| W | 0,6 m% |
| N | 0,3 m% |
| Mn | 0,4 m% |
| Nb | 0,45 m% |

3. Verwendung einer Kobalt-Basis-Legierung nach einem der vorhergehenden Ansprüche für die Herstellung von Implantaten und Prothesen in der Chirurgie und der Zahnheilkunde.
